# EUROPÄISCHE PATENTSCHRIFT

(11) **EP 1 663 988 B1**
(45) Veröffentlichungstag und Bekanntmachung des Hinweises auf die Patenterteilung: **16.04.2014**
(21) Anmeldenummer: 04764195.6
(22) Anmeldetag: 17.08.2004
(51) Int. Cl.: C07D 235/30, C07D 417/12, C07D 405/06, C07D 401/14, A61K 31/4184

(54) **BENZYL-BENZIMIDAZOLYLDERIVATE**
BENZYL-BENZIMIDAZOLYL DERIVATIVES
DERIVES DE BENZYL-BENZIMIDAZOLYLE

(30) Priorität: 12.09.2003 DE 10342503
(43) Veröffentlichungstag der Anmeldung: 07.06.2006
(73) Patentinhaber: Merck Patent GmbH, 64293 Darmstadt (DE)
(72) Erfinder: STÄHLE, Wolfgang, 55218 Ingelheim (DE); JONCZYK, Alfred, 64295 Darmstadt (DE); RAUTENBERG, Wilfried, 64354 Reinheim (DE)
(86) Internationale Anmeldenummer: PCT/EP2004/009205
(87) Internationale Veröffentlichungsnummer: WO 2005/028448

(56) Entgegenhaltungen:
- EP-A- 0 545 845
- EP-A- 0 604 353
- WO-A-00/01676
- WO-A-00/66112
- WO-A-01/25238
- WO-A-02/44156
- WO-A-03/074515
- US-A- 3 941 788
- US-A- 4 004 016
- US-A1- 2002 082 280
- PORCARI, ANTHONY R. ET AL: "Design, Synthesis, and Antiviral Evaluations of 1-(Substituted benzyl)-2-substituted-5,6-dichlorobenzimid azoles as Nonnucleoside Analogs of 2,5,6-Trichloro-1-(.beta.-D-ribofuranosyl) benzimidazole" JOURNAL OF MEDICINAL CHEMISTRY , 41(8), 1252-1262 CODEN: JMCMAR; ISSN: 0022-2623, 1998, XP002302493
- DATABASE CAPLUS [Online] CHEMICAL ABSTRACTS SERVICE, COLUMBUS, OHIO, US; VLAOVIC, DJORDJE ET AL: "Synthesis, antibacterial, and antifungal activities of some new benzimidazoles" retrieved from STN Database accession no. 1992:247903 XP002302494 & BIOSCIENCE, BIOTECHNOLOGY, AND BIOCHEMISTRY , 56(2), 199-206 CODEN: BBBIEJ; ISSN: 0916-8451, 1992,
- DATABASE CAPLUS [Online] CHEMICAL ABSTRACTS SERVICE, COLUMBUS, OHIO, US; TAKAMURA, MASAHIRO ET AL: "Preparation of N-heteroarylthiourea derivatives as endothelin-converting enzyme inhibitors" retrieved from STN Database accession no. 2000:715593 XP002302495 & JP 2000 281659 A2 (SUMITOMO PHARMACEUTICALS CO., LTD., JAPAN) 10. Oktober 2000 (2000-10-10)
- DATABASE CAPLUS [Online] CHEMICAL ABSTRACTS SERVICE, COLUMBUS, OHIO, US; LIU, DING Y. ET AL: "Induction and inhibition of the in vitro N1-oxidation of 9-benzyladenine and isomeric 9-(nitrobenzyl)adenines" retrieved from STN Database accession no. 1999:168997 XP002302496 & DRUG METABOLISM AND DRUG INTERACTIONS , 15(2-3), 141-157 CODEN: DMDIEQ; ISSN: 0792-5077, 1999,
- DATABASE CAPLUS [Online] CHEMICAL ABSTRACTS SERVICE, COLUMBUS, OHIO, US; HAQUE, M. REZAUL ET AL: "Ambident heterocyclic reactivity: alkylation of 2-substituted 4-methylbenzimidazoles" retrieved from STN Database accession no. 1994:507684 XP002302497 & TETRAHEDRON , 50(18), 5535-54 CODEN: TETRAB; ISSN: 0040-4020, 1994,
- DATABASE CAPLUS [Online] CHEMICAL ABSTRACTS SERVICE, COLUMBUS, OHIO, US; CHAUHAN, P. M. S. ET AL: "Synthesis of bis(1-alkyl-2-substituted benzimidazol-5-yl) ketones and their filaricidal activity" retrieved from STN Database accession no. 1988:204554 XP002302498 & INDIAN JOURNAL OF CHEMISTRY, SECTION B: ORGANIC CHEMISTRY INCLUDING MEDICINAL CHEMISTRY , 26B(7), 647-51 CODEN: IJSBDB; ISSN: 0376-4699, 1987,
- DATABASE CAPLUS [Online] CHEMICAL ABSTRACTS SERVICE, COLUMBUS, OHIO, US; POZHARSKII, A. F. ET AL: "Factors affecting the autoxidation of 2-aminobenzimidazoles to 2-nitrobenzimidazole" retrieved from STN Database accession no. 1977:535191 XP002302499 & TEZISY VSES. SOVESHCH. KHIM. NITROSOEDINENII, 5TH , 57-8 PUBLISHER: "NAUKA", MOSCOW, USSR. CODEN: 35DYAT, 1974,
- DATABASE CAPLUS [Online] CHEMICAL ABSTRACTS SERVICE, COLUMBUS, OHIO, US; POZHARSKII, A. F. ET AL: "o-Dimethoxy effect in a Chichibabin reaction" retrieved from STN Database accession no. 1972:153676 XP002302500 & KHIMIYA GETEROTSIKLICHESKIKH SOEDINENII , 7(8), 1105-11 CODEN: KGSSAQ; ISSN: 0132-6244, 1971,
- DATABASE CAPLUS [Online] CHEMICAL ABSTRACTS SERVICE, COLUMBUS, OHIO, US; POZHARSKII, A. F. ET AL: "Benzimidazole derivatives. XXII. Benzimidazole derivatives with electron-donor substituents in the Chichibabin reaction" retrieved from STN Database accession no. 1971:448982 XP002302501 & KHIMIYA GETEROTSIKLICHESKIKH SOEDINENII , (8), 1060-5 CODEN: KGSSAQ; ISSN: 0132-6244, 1970,
- DATABASE CAPLUS [Online] CHEMICAL ABSTRACTS SERVICE, COLUMBUS, OHIO, US; MOKRUSHINA, G. A. ET AL: "Benzo- and naphthazole series. XXVIII. Synthesis of 1-alkyl-2-hydrazinobenzimidazole derivatives" retrieved from STN Database accession no. 1971:76370 XP002302502 & KHIMIYA GETEROTSIKLICHESKIKH SOEDINENII , (10), 1397-400 CODEN: KGSSAQ; ISSN: 0132-6244, 1970,

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Kinasen, insbesondere der Tyrosinkinasen eine Rolle spielt, ferner pharmazeutische Zusammensetzungen, die diese Verbindungen enthalten, sowie die Verwendung der Verbindungen zur Behandlung von kinasebedingter Krankheiten.

Im einzelnen betrifft die vorliegende Erfindung Verbindungen, die die Signaltransduktion der Tyrosinkinasen hemmen, regulieren und/oder modulieren, Zusammensetzungen, die diese Verbindungen enthalten, sowie Verfahren zu ihrer Verwendung zur Behandlung von tyrosinkinasebedingten Krankheiten und Leiden wie Angiogenese, Krebs, Tumorwachstum, Arteriosklerose, altersbedingte Makula-Degeneration, diabetische Retinopathie, Entzündungserkrankungen und dergleichen bei Säugetieren.
Bei den Tyrosinkinasen handelt es sich um eine Klasse von Enyzmen, die die Übertragung des endständigen Phosphats des Adenosintriphosphats auf Tyrosinreste bei Proteinsubstraten katalysieren. Man nimmt an, dass den Tyrosinkinasen bei verschiedenen Zeilfunktionen über die Substratphosphorylierung eine wesentliche Rolle bei der Signaltransduktion zukommt. Obwohl die genaue Mechanismen der Signaltransduktion noch unklar sind, wurde gezeigt, dass die Tyrosinkinasen wichtige Faktoren bei der Zellproliferation, der Karzinogenese und der Zelldifferenzierung darstellen.
Die Tyrosinkinasen lassen sich in Rezeptor-Tyrosinkinasen und zytosolische Tyrosinkinasen einteilen. Die Rezeptor-Tyrosinkinasen weisen einen extrazellulären Teil, einen Transmembranteil und einen intrazellulären Teil auf, während die zytosolischen Tyrosinkinasen ausschließlich intrazellulär vorliegen.
Die Rezeptor-Tyrosinkinasen bestehen aus einer Vielzahl von Transmembranrezeptoren mit unterschiedlicher biologischer Wirksamkeit. So wurden ungefähr 20 verschiedene Unterfamilien von Rezeptor-Tyrosinkinasen identifiziert. Eine Tyrosinkinase-Unterfamilie, die die Bezeichnung HER-Unterfamilie trägt, besteht aus EGFR, HER2, HER3 und HER4. Zu den Liganden dieser Rezeptor-Unterfamilie zählen der Epithel-Wachstumsfaktor, TGF-α, Amphiregulin, HB-EGF, Betacellulin und Heregulin. Die Insulin-Unterfamilie, zu der INS-R, IGF-IR und IR-R zählen, stellt eine weitere Unterfamilie dieser Rezeptor-Tyrosinkinasen dar. Die PDGF-Unterfamilie beinhaltet den PDGF-α- and -β-Rezeptor, CSFIR, c-kit und FLK-II. Außerdem gibt es die FLK-Familie, die aus dem Kinaseinsertdomänenrezeptor (KDR), der fötalen Leberkinase-1 (FLK-1), der fötalen Leberkinase-4 (FLK-4) und der fms-Tyrosinkinase-1 (flt-1) besteht. Die PDGF- und FLK-Familie werden üblicherweise aufgrund der zwischen den beiden Gruppen bestehenden Ähnlichkeiten gemeinsam diskutiert. Für eine genaue Diskussion der Rezeptor-Tyrosinkinasen siehe die Arbeit von Plowman et al., DN & P 7(6):334-339, 1994, die hiermit durch Bezugnahme aufgenommen wird.
Die zytosolischen Tyrosinkinasen bestehen ebenfalls aus einer Vielzahl von Unterfamilien, darunter Src, Frk, Btk, Csk, Abl, Zap70, Fes/Fps, Fak, Jak, Ack, and LIMK. Jede dieser Unterfamilien ist weiter in verschiedene Rezeptoren unterteilt. So stellt zum Beispiel die Src-Unterfamilie eine der größten Unterfamilien dar. Sie beinhaltet Src, Yes, Fyn, Lyn, Lck, Blk, Hck, Fgr und Yrk. Die Src-Enzymunterfamilie wurde mit der Onkogenese in Verbindung gebracht. Für eine genauere Diskussion der zytosolischen Tyrosinkinasen, siehe die Arbeit von Bolen Oncogene, 8:2025-2031 (1993), die hiermit durch Bezugnahme aufgenommen wird.
Sowohl die Rezeptor-Tyrosinkinasen als auch die zytosolischen Tyrosinkinasen sind an Signalübertragungswegen der Zelle, die zu verschiedenen Leidenszuständen führen, darunter Krebs, Schuppenflechte und Hyperimmunreaktionen, beteiligt.
Es wurde vorgeschlagen, dass verschiedene Rezeptor-Tyrosinkinasen sowie die an sie bindenden Wachstumsfaktoren eine Rolle bei den Angiogenese spielen, obwohl einige die Angiogenese indirekt fördern könnten (Mustonen und Alitalo, J. Cell Biol. 129:895-898, 1995). Eine dieser Rezeptor-Tyrosinkinasen ist die fötale Leberkinase 1, auch FLK-1 genannt. Das menschliche Analog der FLK-1 ist der kinase-insertdomänenhaltige Rezeptor KDR, der auch unter der Bezeichnung Gefäßendothelzellenwachstumsfaktorrezeptor 2 bzw. VEGFR-2 bekannt ist, da er VEGF hochaffin bindet. Schließlich wurde die Maus-Version dieses Rezeptors auch ebenfalls NYK genannt (Oelrichs et al., Oncogene 8(1):11-15, 1993). VEGF und KDR stellen ein Ligand-Rezeptor-Paar dar, das eine wesentliche Rolle bei der Proliferation der Gefäßendothelzellen und der Bildung und Sprossung der Blutgefäße, die als Vaskulogenese bzw. Angiogenese bezeichnet werden, spielt.
Die Angiogenese ist durch eine übermäßig starke Aktivität des Gefäßendothelwachstumsfaktors (VEGF) gekennzeichnet. Der VEGF besteht eigentlich aus einer Familie von Liganden (Klagsburn und D'Amore, Cytokine & Growth Factor Reviews 7:259-270, 1996). Der VEGF bindet den hochaffinen transmembranösen Tyrosinkinaserezepzor KDR und die verwandte fms-Tyrosinkinase-1, auch unter der Bezeichnung Flt-1 oder Gefäßendothelzellenwachstumsfaktorrezeptor 1 (VEGFR-1) bekannt. Aus Zellkultur- und Gen- Knockout-Versuchen geht hervor, dass jeder Rezeptor zu unterschiedlichen Aspekten der Angiogenese beiträgt. Der KDR führt die mitogene Funktion des VEGF herbei, während Flt-1 nichtmitogene Funktionen, wie diejenigen, die mit der Zelladhäsion in Zusammenhang stehen, zu modulieren scheint. Eine Hemmung des KDR moduliert daher das Niveau der mitogenen VEGF-Aktivität. Tatsächlich wurde gezeigt, dass das Tumorwachstum von der antiangiogenen Wirkung der VEGF-Rezeptor-Antagonisten beeinflusst wird (Kim et al., Nature 362, S. 841- 844, 1993).
Feste Tumore können daher mit Tyrosinhemmern behandelt werden, da diese Tumore für die Bildung der zur Unterstützung ihres Wachstums erforderlichen Blutgefäße auf Angiogenese angewiesen sind. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom. Zu weiteren Beispielen zählen Karzinome, bei denen eine Überexpression oder Aktivierung von Rafaktivierenden Onkogenen (z.B. K-ras, erb-B) beobachtet wird. Zu diesen Karzinomen zählen Bauchspeicheldrüsen- und Brustkarzinom. Hemmstoffe dieser Tyrosinkinasen eignen sich daher zur Vorbeugung und Behandlung von proliferativen Krankheiten, die durch diese Enzyme bedingt sind.
Die angiogene Aktivität des VEGF ist nicht auf Tumore beschränkt. Der VEGF ist für die bei diabetischer Retinopathie in bzw. in der Nähe der Retina produzierte angiogene Aktivität verantwortlich. Dieses Gefäßwachstum in der Retina führt zu geschwächter Sehkraft und schließlich Erblindung. Die VEGF-mRNA- und -protein-Spiegel im Auge werden durch Leiden wie Netzhautvenenokklusion beim Primaten sowie verringertem pO₂-Spiegel bei der Maus, die zu Gefäßneubildung führen, erhöht. Intraokular injizierte monoklonale Anti-VEGF-Antikörper, oder VEGF-Rezeptor-Immunkonjugate, hemmen sowohl im Primaten- als auch im Nagetiermodell die Gefäßneubildung im Auge. Unabhängig vom Grund der Induktion des VEGF bei der diabetischen Retinopathie des Menschen, eignet sich die Hemmung des Augen-VEGF zur Behandlung dieser Krankheit.
Die VEGF-Expression ist auch in hypoxischen Regionen von tierischen und menschlichen Tumoren neben Nekrosezonen stark erhöht. Der VEG F wird außerdem durch die Expression der Onkogene ras, raf, src und p53-Mutante (die alle bei der Bekämpfung von Krebs von Bedeutung sind) hinaufreguliert. Monoklonale Anti-VEGF-Antikörper hemmen bei der Nacktmaus das Wachstum menschlicher Tumore. Obwohl die gleichen Tumorzellen in Kultur weiterhin VEGF exprimieren, verringern die Antikörper ihre Zellteilungsrate nicht. So wirkt der aus Tumoren stammende VEGF nicht als autokriner mitogener Faktor. Der VEGF trägt daher in vivo dadurch zum Tumorwachstum bei, dass er durch seine parakrine Gefäßendothelzellen-Chemotaxis- und -Mitogeneseaktivität die Angiogenese fördert. Diese monoklonalen Antikörper hemmen auch das Wachstum von typischerweise weniger stark vaskularisierten Human-Kolonkarzinomen bei thymuslosen Mäusen und verringern die Anzahl der aus inokulierten Zellen entstehenden Tumore.
Die Expression eines VEGF-bindenden Konstrukts von Flk-1, Flt-1, dem zur Entfernung der zytoplasmatischen Tyrosinkinasedomänen, jedoch unter Beibehaltung eines Membranankers, verkürzten Maus-KDR-Rezeptorhomologs, in Viren stoppt praktisch das Wachstum eines transplantierbaren Glioblastoms bei der Maus, vermutlich aufgrund des dominant-negativen Mechanismus der Heterodimerbildung mit transmembranösen Endothelzellen-VEGF-Rezeptoren. Embryostammzellen, die in der Nacktmaus üblicherweise in Form von festen Tumoren wachsen, bilden bei Knock-out aller beider VEGF-Allele keine nachweisbaren Tumore. Aus diesen Daten gemeinsam geht die Rolle des VEGF beim Wachstum fester Tumore hervor. Die Hemmung von von KDR bzw. Flt-1 ist an der pathologischen Angiogenese beteiligt, und diese Rezeptoren eignen sich zur Behandlung von Krankheiten, bei denen Angiogenese einen Teil der Gesamtpathologie, z.B. Entzündung, diabetische Retina-Vaskularisierung sowie verschiedene Formen von Krebs, darstellt, da bekannt ist, dass das Tumorwachstum angiogeneseabhängig ist (Weidner et al., N. Engl. J. Med., 324, S. 1-8, 1991).

Bei Angiopoieten 1 (Ang1), einem Liganden für die endothelspezifische Rezeptor-Tyrosinkinase TIE-2, handelt es sich um einen neuen angiogenen Faktor (Davis et al, Cell, 1996, 87:1161-1169; Partanen et al, Mol. Cell Biol., 12:1698-1707 (1992); US-Patent Nr. 5,521,073; 5,879,672; 5,877,020; und 6,030,831). Das Akronym TIE steht für "Tyrosinkinase mit lg- und EGF-Homologiedomänen". TIE wird zur Identifizierung einer Klasse von Rezeptor-Tyrosinkinasen verwendet, die ausschließlich in Gefäßendothelzellen und frühen hämopoietischen Zellen exprimiert werden. TIE-Rezeptorkinasen sind typischerweise durch das Vorhandensein einer EGF-ähnlichen Domäne und einer Immunglobulin (IG)-ähnlichen Domäne charakterisiert, die aus extrazellulären Faltungseinheiten, die durch Disulfidbrückenbindungen zwischen den Ketten stabilisiert sind, besteht (Partanen et al Curr. Topics Microbiol. Immunol., 1999, 237:159-172). Im Gegensatz zu VEGF, der seine Funktion während der frühen Stadien in der Gefäßentwicklung ausübt, wirken Ang1 und sein Rezeptor TIE-2 während der späteren Stadien in der Gefäßentwicklung, d.h. während der Gefäßumbildung (Umbildung bezieht sich auf die Bildung eines Gefäßlumens) und Reifung (Yancopoulos et al, Cell, 1998, 93:661-664; Peters, K.G., Circ. Res., 1998, 83(3):342-3; Suri et al, Cell, 87, 1171-1180 (1996)).

Demzufolge würde man erwarten, daß eine Hemmung von TIE-2 die Umbildung und Reifung eines durch Angiogenese initiierten neuen Gefäßsystems und dadurch den Angiogeneseprozeß unterbrechen sollte. Weiterhin würde eine Hemmung an der Kinasedomäne-Bindungsstelle von VEGFR-2 die Phosphorylierung von Tyrosinresten blockieren und dazu dienen, die Initiation der Angiogenese zu unterbrechen. Daher darf man annehmen, daß die Hemmung von TIE-2 und/oder VEGFR-2 die Tumorangiogenese verhindern und dazu dienen sollte, das Tumorwachstum zu verlangsamen oder vollständig zu beseitigen. Dementsprechend könnte man eine Behandlung von Krebs und anderen mit unangemessener Angiogenese einhergehenden Erkrankungen bereitstellen.

Die vorliegende Erfindung richtet sich auf Verfahren zur Regulation, Modulation oder Hemmung der TIE-2 zur Vorbeugung und/oder Behandlung von Erkrankungen im Zusammenhang mit unregulierter oder gestörter TIE-2-Aktivität. Insbesondere lassen sich die erfindungsgemäßen Verbindungen auch bei der Behandlung gewisser Krebsformen einsetzen. Weiterhin können die erfindungsgemäßen Verbindungen verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können erfindungsgemäße Verbindungen zur Isolierung und zur Untersuchung der Aktivität oder Expression von TIE-2 verwendet werden. Außerdem eigenen sie sich insbesondere zur Verwendung in diagnostischen Verfahren zu Erkrankungen im Zusammenhang mit unregulierter oder gestörter TIE-2-Aktivität.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279 oder Dancey und Sausville Nature Drug Discovery, 2003, 2, 296-313).

Die Identifikation von kleinen Verbindungen, die die Signaltransduktion der Tyrosinkinasen spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Insbesondere zeigen sie inhibierende Eigenschaften bei Tyrosinkinasen.

Wie hierin besprochen, sind diese Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind. Gegenstand der vorliegenden Erfindung sind deshalb erfindungsgemäße Verbindungen als Promotoren oder Inhibitoren, bevorzugt als Inhibitoren der hierin beschriebenen Signalwege. Bevorzugter Gegenstand der Erfindung sind deshalb erfindungsgemäße Verbindungen als Promotoren oder Inhibitoren, bevorzugt als Inhibitoren von Tyrosinkinase abhängigen Signal-Übertragungswege. Ein bevorzugter Gegenstand der Erfindung sind deshalb erfindungsgemäße Verbindungen als Promotoren oder Inhibitoren, bevorzugt als Inhibitoren von TIE-2, VEGFR, PDGFR, FGFR und/oder FLT/KDR.

In diesem Zusammenhang werden Psoriasis, Arthritis, Entzündungen, Endometriose, Vernarbung, gutartige Prostatahyperplasie, immunologische Krankheiten, Autoimmunkrankheiten und Immunschwächekrankheiten als nicht krebsartige Krankheiten angesehen, von denen Arthritis, Entzündung, immunologische Krankheiten, Autoimmunkrankheiten und Immunschwächekrankheiten gewöhnlich als nicht hyperproliferative Erkrankungen angesehen werden. In diesem Zusammenhang sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich als hyperproliferative Erkrankungen angesehen werden. Insbesondere krebsartiges Zellwachstum und insbesondere durch Raf-Kinase vermitteltes krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., unmittelbar vor der Veröffentlichung, Manuskript BJ20020786).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt, Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die erfindungsgemäßen Verbindungen eignen sich auch als p38 Kinaselnhibitoren.

Andere Heteroarylharnstoffe, die p38 Kinase inhibieren sind in der WO 02/85859 beschrieben.

### STAND DER TECHNIK

In der WO 02/44156 sind andere Benzimidazol-Derivate als TIE-2 und/oder VEGFR2-Inhibitoten beschrieben.

### ZUSAMMENFASSUNG DER ERFINDUNG

Erfindung ausgewählt aus der Gruppe
3-((2-Amino-1-benzyl-1H-benzimidazol-5-yl)-propionsäure,
3-((2-Amino-1-biphenyl-4-ylmethyl-1H-benzimidazol-5-yl)-propionsäure,
3-[2-Amino-1-(4-methoxy-benzyl)-1H-benzimidazol-5-yl]-propionsäure,
3-[2-Amino-1-(2-methoxy-benzyl)-1H-benzimidazol-5-yl]-propionsäure,
3-[2-Amino-1-(3-methoxy-benzyl)-1H-benzimidazol-5-yl]-propionsäure,
3-[2-Amino-1-(4-chlor-benzyl)-1H-benzimidazol-5-yl]-propionsäure,
3-[2-Amino-1-(4-methyl-benzyl)-1H-benzimidazol-5-yl]-propionsäure,
3-[2-Amino-1-(3-methyl-benzyl)-1H-benzimidazol-5-yl]-propionsäure,
3-[2-Amino-1-(3-chlor-benzyl)-1H-benzimidazol-5-yl]-propionsäure,
3-((2-amino-1-benzo[1,3]dioxol-5-ylmethyl-1H-benzimidazol-5-yl)-propionsäure,
3-((2-Amino-1-biphenyl-4-ylmethyl-1H-benzimidazol-5-yl)-propan-1-ol,
3-[2-Amino-1-(4-methoxy-benzyl)-1H-benzimidazol-5-yl]-propan-1-ol,
3-[2-Amino-1-(2-methoxy-benzyl)-1H-benzimidazol-5-yl]-propan-1-ol,
3-[2-Amino-1-(3-methoxy-benzyl)-1H-benzimidazol-5-yl]-propan-1-ol,
3-[2-Amino-1-(4-chlor-benzyl)-1H-benzimidazol-5-yl]-propan-1-ol,
3-[2-Amino-1-(4-methyl-benzyl)-1H-benzimidazol-5-yl]-propan-1-ol,
3-[2-Amino-1-(3-methyl-benzyl)-1H-benzimidazol-5-yl]-propan-1-ol,
3-[2-Amino-1-(3-chlor-benzyl)-1H-benzimidazol-5-yl]-propan-1-ol,
3-((2-Amino-1-benzo[1,3]dioxol-5-ylmethyl-1H-benzimidazol-5-yl)-propan-1-ol,
3-[2-Amino-1-(3-methoxy-benzyl)-1H-benzimidazol-5-yl]-propionsäure methyl ester,
3-[2-Amino-1-(4-chlor-benzyl)-1H-benzimidazol-5-yl]-propionsäure methyl ester,
3-[2-Amino-1-(3-methyl-benzyl)-1 H-benzimidazol-5-yl]-propionsäure methyl ester,
3-((2-Amino-1-biphenyl-4-ylmethyl-1H-benzimidazol-5-yl)-propionamid,
3-[2-Amino-1-(4-chlor-benzyl)-1H-benzimidazol-5-yl]-propionamid
3-((2-Amino-1-benzo[1,3]dioxol-5-ylmethyl-1H-benzimidazol-5-yl)-propionamid,
3-[2-Amino-1-(3-methyl-benzyl)-1H-benzimidazol-5-yl]-propionamid,
3-{2-Amino-1-[4-(2,3-dichlor-benzolsulfonylamino)-benzyl]-1H-benzimidazol-5-yl}-propionsäure,
1-Benzyl-5-morpholin-4-ylmethyl-1H-benzimidazol-2-ylamine,
N-{4-[2-Amino-5-(3-hydroxy-propyl)-benzimidazol-1-ylmethyl]-phenyl}-2,3-dichlor-benzolsulfonamid,
1-((4-Chlor-benzyl)-5-morpholin-4-ylmethyl-1H-benzimidazol-2-ylamin,
1-Benzyl-5-(4-phenyl-piperazin-1-ylmethyl)-1H-benzimidazol-2-ylamin,
5-[4-(5-Methyl-1H-imidazol-4-yl)-piperidin-1-ylmethyl]-1-(3-trifluoromethyl-benzyl)-1H-benzimidazol-2-ylamin,
5-((4-Benzo[1,2,5]thiadiazol-5-yl-plperazin-1-ylmethyl)-1-(3-trifluoromethyl-benzyl)-1 H-benzimidazol-2-ylamin,
3-[2-Amino-1-(4-amino-benzyl)-1H-benzimidazol-5-yl]-propan-1-ol,
2-Amino-1-(3,5-difluor-benzyl)-1H-benzimidazol-5-carbon säure,
2-Amino-1-(4-methanesulfonyl-benzyl)-1H-benzimidazol-5-carbonsäure,
2-Amino-1-(4-fluor-benzyl)-1H-benzimidazol-5-carbonsäure,
sowie ihre Solvate und Stereoisomere, einschließlich deren Mischungen in allen Verhältnissen.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomere Verbindungen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Die Verbindungen aus Anspruch 1 können vorzugsweise erhalten werden, indem man Anilinderivate der Formel II (J. Med Chem. 1992, 35, Seite 877-885, THL 2000, 41, Seote 9871-9874) mit Bromcyan umsetzt.

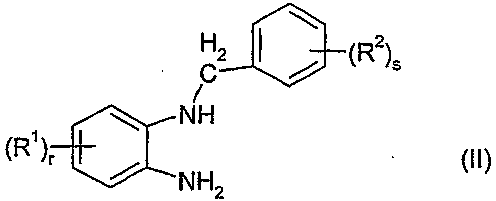

Die Umsetzung erfolgt nach Methoden, die dem Fachmann bekannt sind. Zunächst erfolgt Reaktion in einem geeigneten Lösungsmittel, gegebenenfalls in Gegenwart einer organischen Base, wie z.B. Triethylamin oder einer anorganischen Base, wie z.B. eines Alkali- oder Erdalkalicarbonats.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohienstoff, Chlorform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Eine Base der erfindungsgemäßen Verbindungen der Formel kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der erfindungsgemäßen Verbindungen verwendet werden.

Andererseits können Verbindungen der Formel I mit Basen (z.B. Natriumoder Kaliumhydroxid oder -carbonat) in die entsprechenden Metall-, insbesondere Alkalimetall- oder Erdalkalimetall- oder in die entsprechenden Ammoniumsalze umgewandelt werden.

Auch physiologisch unbedenkliche organische Basen, wie z.B. Ethanolamin können verwendet werden.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Satz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten

Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen.

Liposomen können aus verschiedenen Phospholipiden, wie z.B. Cholesterin, Stearylamin oder Phosphatidylcholinen, gebildet werden.

Die erfindungsgemäßen Verbindungen sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine erfindungsgemäße Verbindung und/oder ihre pharmazeutisch verwendbaren Derivate, Solvate und Stereoisomere, einschließlich deren Mischungen in allen Verhältnissen, und mindestens einen weiteren Arzneimitteiwirkstoff.

Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren Derivate, Solvate und Stereoisomere, einschließlich deren Mischungen in allen Verhältnissen,
   und
(b) einer wirksamen Menge eines weiteren Arzneimitteiwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.
Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die Verwendung von erfindungsgemäßen Verbindungen nach Anspruch 1 und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung derverfindungsgemäßen Verbindungen nach Anspruch 1 und/oder ihre physiologisch unbedenklichen Salze und Solvate zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.
Die vorliegende Erfindung umfasst auch die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.

Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die erfindungsgemäßen Verbindungen nach Anspruch 1 können an Patienten zur Behandlung von Krebs verabreicht werden. Die vorliegenden Verbindungen hemmen die Tumorangiogenese und beeinflussen so das Wachstum von Tumoren (J. Rak et al. Cancer Research, 55:4575-4580, 1995). Die angiogenesehemmenden Eigenschaften der vorliegenden Verbindungen nach Anspruch 1 eignen sich auch zur Behandlung bestimmter Formen von Blindheit, die mit Retina-Gefäßneubildung in Zusammenhang stehen.
Die Verbindungen nach Anspruch 1 eignen sich auch zur Behandlung bestimmter Knochen-Pathologien wie Osteosarkom, Osteoarthritis und Rachitis, die auch unter der Bezeichnung onkogene Osteomalazie bekannt ist (Hasegawa et al., Skeletal Radiol. 28, S.41-45, 1999; Gerber et al., Nature Medicine, Bd. 5, Nr. 6, S.623-628, Juni 1999). Da der VEGF durch den in reifen Osteoklasten exprimierten KDR/Flk-1 direkt die osteoklastische Knochenresorption fördert (FEBS Let. 473:161-164 (2000); Endocrinology, 141:1667 (2000)), eignen sich die vorliegenden Verbindungen auch zur Behandlung und Vorbeugung von Leiden, die mit Knochenresorption in Zusammenhang stehen, wie Osteoporose und Morbus Paget.

Die Verbindungen können dadurch, dass sie zerebrale Ödeme, Gewebeschädigung und ischämiebedingte Reperfusionsverletzungen reduzieren, auch zur Verringerung oder Vorbeugung von Gewebeschäden, die nach zerebralen ischämischen Ereignissen wie Gehirnschlag auftreten, verwendet werden (Drug News Perspect 11:265-270 (1998); J. Clin. Invest. 104:1613-1620 (1999)).

Gegenstand der Erfindung ist somit die Verwendung von Verbindungen nach Anspruch 1, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt sind hierbei Kinasen ausgewählt aus der Gruppe der Tyrosinkinasen.

Vorzugsweise handelt es sich bei den Tyrosinkinasen um TIE-2, VEGFR, PDGFR, FGFR und/oder FLT/KDR.

Bevorzugt ist die Verwendung von Verbindungen gemäß Anspruch 1, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, die durch Inhibierung der Tyrosinkinasen durch die Verbindungen nach Anspruch 1 beeinflußt werden.

Besonders bevorzugt ist die Verwendung zur Herstellung eines Arzneimittels zur Behandlung von Krankheiten, die durch Inhibierung von TIE-2, VEGFR, PDGFR, FGFR und/oder FLT/KDR durch die Verbindungen nach Anspruch 1 beeinflußt werden.

Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren des Plattenepithel, der Blasen, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge.

Der feste Tumor ist weiterhin vorzugsweise ausgewählt aus der Gruppe Monozytenleukämie, Lungenadenokarzinom, kleinzellige Lungenkarzinome, Bauchspeicheldrüsenkrebs, Glioblastome und Brustkarzinom.

Gegenstand der Erfindung ist weiterhin die Verwendung der erfindungsgemäßen Verbindungen zur Behandlung einer Krankheit, an der Angiogenese beteiligt ist.

Vorzugsweise handelt es sich bei der Krankheit um eine Augenkrankheit.

Gegenstand der Erfindung ist weiterhin die Verwendung zur Behandlung von Retina-Vaskularisierung, diabetischer Retinopathie, altersbedingter Makula-Degeneration und/oder Entzündungskrankheiten.

Die Entzündungskrankheit ist vorzugsweise ausgewählt aus der Gruppe rheumatoide Arthritis, Schuppenflechte, Kontaktdermatitis und Spät-Typ der Überempfindlichkeitsreaktion stammt.

Gegenstand der Erfindung ist weiterhin die Verwendung der erfindungsgemäßen Verbindungen zur Behandlung von Knochen-Pathologien, wobei die Knochenpathologie aus der Gruppe Osteosarkom, Osteoarthritis und Rachitis stammt.

Hierbei handelt es sich um Krebserkrankungen oder nicht krebsartige Erkrankungen.
Die nicht krebsartigen Erkrankungen sind ausgewählt aus der Gruppe bestehend aus Psoriasis, Arthritis, Entzündungen, Endometriose, Vernarbung, gutartiger Prostatahyperplasie, immunologischer Krankheiten, Autoimmunkrankheiten und Immunschwächekrankheiten.

Die krebsartigen Erkrankungen sind ausgewählt aus der Gruppe bestehend aus Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, chronischer Leukämie und akuter Leukämie.

Die erfindungsgemäßen Verbindungen können auch gemeinsam mit anderen gut bekannten Therapeutika, die aufgrund ihrer jeweiligen Eignung für das behandelte Leiden ausgewählt werden, verabreicht werden. So wären zum Beispiel bei Knochenleiden Kombinationen günstig, die antiresorptiv wirkende Bisphosphonate, wie Alendronat und Risedronat, Integrinblocker (wie sie weiter unten definiert werden), wie avβ3-Antagonisten, bei der Hormontherapie verwendetete konjugierte Östrogene wie Prempro®, Premarin® und Endometrion®; selektive Östrogenrezeptormodulatoren (SERMs) wie Raloxifen, Droloxifen, CP-336,156 (Pfizer) und Lasofoxifen, Kathepsin-K-Hemmer und ATP-Protonenpumpenhemmer enthalten.
Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxylphenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.

Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron; Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN 10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Amino-propylamino)-7,10-dihydroxy-2-(2-hydroxyethylaminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diaza-tetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549). VEGF-Rezeptorkinase-Assay
Die VEGF-Rezeptorkinaseaktivität wird durch Einbau von radioaktiv markiertem Phosphat in 4:1 Polyglutaminsäure/Tyrosin-Substrat (pEY) bestimmt. Das phosphorylierte pEY-Produkt wird auf einer Filtermembran festgehalten, und der Einbau des radioaktiv markierten Phosphats wird durch Szintillationszählung quantitativ bestimmt.

### MATERIALIEN

### VEGF-Rezeptorkinase

Die intrazelluläre-Tyrosinkinase-Domänen des menschlichen KDR (Terman, B. I. et al. Oncogene (1991) Bd. 6, S. 1677-1683.) und Flt-1 (Shibuya, M. et al. Oncogene (1990) Bd. 5, S. 519-524) wurden als Glutathion-S-transferase (GST)-Genfusionsproteine kloniert. Dies geschah durch Klonieren der Zytoplasma-Domäne der KDR-Kinase als leserastergerechte Fusion am Carboxy-Terminus des GST-Gens. Die löslichen rekombinanten GST-Kinasedomäne-Fusionsproteine wurden in *Spodoptera frugiperda* (Sf21) Insektenzellen (Invitrogen) unter Verwendung eines Baculovirus-Expressionsvektors (pAcG2T, Pharmingen) exprimiert.

### Lysepuffer

50 mM Tris pH 7,4, 0,5 M NaCl, 5 mM DTT, 1 mM EDTA, 0,5% Triton X-100, 10% Glycerin, je 10 mg/ml Leupeptin, Pepstatin und Aprotinin sowie 1 mM Phenylmethylsulfonylfluorid (alle von Sigma).

### Waschpuffer

50 mM Tris pH 7,4, 0,5 M NaCl, 5 mM DTT, 1 mM EDTA, 0.05% Triton X-100, 10% Glycerin, je 10 mg/ml Leupeptin, Pepstatin und Aprotinin sowie 1 mM Phenylmethylsulfonylfluorid.

### Dialysepuffer

50 mM Tris pH 7,4, 0,5 M NaCl, 5 mM DTT, 1 mM EDTA, 0.05% Triton X-100, 50% Glycerin, je 10 mg/ml Leupeptin, Pepstatin und Aprotinin sowie 1 mM Phenylmethylsulfonylfluorid.

### 10× Reaktionspuffer

200 mM Tris, pH 7,4, 1,0 M NaCl, 50 mM MnCl₂, 10 mM DTT und 5 mg/ml Rinderserumalbumin [bovine serum albumin = BSA] (Sigma).

### Enzymverdünnungspuffer

50 mM Tris, pH 7,4, 0,1 M NaCl, 1 mM DTT, 10% Glycerin, 100 mg/ml BSA.
10× Substrat
750 µg/ml Poly(glutaminsäure/Tyrosin; 4:1) (Sigma).

### Stopp-Lösung

30% Trichloressigsäure, 0,2 M Natriumpyrophosphat (beide von Fisher). Waschlösung
15% Trichloressigsäure, 0,2 M Natriumpyrophosphat.

### Filterplatten

Millipore #MAFC NOB, GF/C 96-Well-Glasfaserplatte.

### Verfahren A - Proteinaufreinigung

1. Die Sf21-Zellen wurden mit dem rekombinanten Virus bei einer m.o.i. (Multiplizität der Infektion) von 5 Viruspartikeln/Zelle infiziert und 48 Stunden lang bei 27°C gezüchtet.
2. Alle Schritte wurden bei 4°C durchgeführt. Die infizierten Zellen wurden durch Zentrifugieren bei 1000×g geerntet und 30 Minuten bei 4°C mit 1/10 Volumen Lysepuffer lysiert und anschließend 1 Stunde lang bei 100.000×g zentrifugiert. Der Überstand wurde dann über eine mit Lysepuffer äquilibrierte Glutathion-Sepharose-Säure (Pharmacia) gegeben und mit 5 Volumina des gleichen Puffers und anschließend 5 Volumina Waschpuffer gewaschen. Das rekombinante GST-KDR-Protein wurde mit Waschpuffer/10 mM reduziertem Glutathion (Sigma) eluiert und gegen Dialysepuffer dialysiert.

### Verfahren B - VEGF-Rezeptorkinase-Assay

1. Assay mit 5 µl Hemmstoff oder Kontrolle in 50% DMSO versetzen.
2. Mit 35 µl Reaktionsmischung, die 5 µl 10x Reaktionspuffer, 5 µl 25 mM ATP/10 µCi[³³ P]ATP (Amersham) und 5 µl 10x Substrat enthält, versetzen.
3. Reaktion durch Zugabe von 10 µl KDR (25 nM) in Enzymverdünnungspuffer starten.
4. Mischen und 15 Minuten lang bei Raumtemperatur inkubieren.
5. Reaktion durch Zugabe von 50 µl Stopp-Lösung stoppen.
6. 15 Minuten lang bei 4°C inkubieren.
7. 90-µl-Aliquot auf Filterplatte überführen.
8. Absaugen und 3 Mal mit Waschlösung waschen.
9. 30 µl Szintillations-Cocktail zugeben, Platte verschließen und in einem Szintillations-Zähler Typ Wallac Microbeta zählen.

Mitogenese-Assay an menschlichen Nabelschnurvenenendothelzellen Die Expression von VEGF-Rezeptoren, die mitogene Reaktionen auf den Wachstumsfaktor vermitteln, ist größtenteils auf Gefäßendothelzellen beschränkt. Kultivierte menschliche Nabelschnurvenenendothelzellen (HUVECs) proliferieren als Reaktion auf Behandlung mit VEGF und können als Assaysystem zur quantitativen Bestimmung der Auswirkungen von KDR-Kinasehemmern auf die Stimulation des VEGF verwendet werden. In dem beschriebenen Assay werden Einzelzellschichten von HUVECs im Ruhezustand 2 Stunden vor der Zugabe von VEGF oder "basic fibroblast growth factor" (bFGF) mit dem Konstituens oder der Testverbindung behandelt. Die mitogene Reaktion auf VEGF oder bFGF wird durch Messung des Einbaus von [³H]Thymidin in die Zell-DNA bestimmt.

### Materialien

### HUVECs

Als Primärkulturisolate tiefgefrorene HUVECs werden von Clonetics Corp bezogen. Die Zellen werden im Endothel-Wachstumsmedium (Endothelial Growth Medium = EGM; Clonetics) erhalten und in der 3. - 7. Passage für die Mitogenitätsassays verwendet.

### Kulturplatten

NUNCLON 96-Well-Polystyrol-Gewebekulturplattten (NUNC #167008).

### Assay-Medium

Nach Dulbecco modifiziertes Eagle-Medium mit 1 g/ml Glucose (DMEM mit niedrigem Glucosegehalt; Mediatech) plus 10% (v/v) fötales Rinderserum (Clonetics).

### Testverbindungen

Mit den Arbeitsstammlösungen der Testverbindungen wird mit 100% Dimethylsulfoxid (DMSO) solange eine Reihenverdünnung durchgeführt, bis ihre Konzentrationen um das 400-fache höher als die gewünschte Endkonzentration sind. Die letzten Verdünnungen (Konzentration 1 x) werden unmittelbar vor Zugabe zu den Zellen mit Assay-Medium hergestellt.

### 10× Wachstumsfaktoren

Lösungen des menschlichen VEGF 165 (500 ng/ml; R&D Systems) und bFGF (10 ng/ml; R&D Systems) werden mit Assay-Medium hergestellt. 10x [³H]-Thymidin
[Methyl-³H]-Thymidin (20 Ci/mmol; Dupont-NEN) wird mit DMEM-Medium mit niedrigem Glucosegehalt auf 80 µCi/ml verdünnt.

### Zellwaschmedium

Hank's balanced salt solution (Mediatech) mit 1 mg/ml Rinderserurnalbumin (Boehringer-Mannheim).

### Zell-Lyse-Lösung

1 N NaOH, 2% (w/v) Na₂CO₃.

### Verfahren 1

In EGM gehaltene HUVEC-Einzelzellschichten werden durch Trypsinbehandlung geerntet und in einer Dichte von 4000 Zellen pro 100 µl Assay-Medium pro Näpfchen in 96-Well-Platten überimpft. Das Wachstum der Zellen wird 24 Stunden bei 37°C in einer 5% CO₂ enthaltenden feuchten Atmosphäre gestoppt.

### Verfahren 2

Das Wachstumsstoppmedium wird durch 100 µl Assay-Medium ersetzt, das entweder das Konstituens (0,25% [v/v] DMSO) oder die erwünschte Endkonzentration der Testverbindung enthält. Alle Bestimmungen werden in dreifacher Wiederholung durchgeführt. Die Zellen werden dann 2 Stunden bei 37°C/5% CO₂ inkubiert, so dass die Testverbindungen in die Zellen eindringen können.

### Verfahren 3

Nach 2-stündiger Vorbehandlung werden die Zellen durch Zugabe von 10 µl Assay-Medium, 10x VEGF-Lösung oder 10x bFGF-Lösung pro Näpfchen stimuliert. Die Zellen werden dann bei 37°C/5% CO₂ inkubiert.

### Verfahren 4

Nach 24 Stunden in Anwesenheit der Wachstumsfaktoren wird mit 10× [³H]-Thymidin (10 µl/well) versetzt.

### Verfahren 5

Drei Tage nach dem Versetzen mit [³H]-Thymidin wird das Medium abgesaugt und die Zellen werden zweimal mit Zellwaschmedium gewaschen (400 µl/well, anschließend 200 µl/well). Die gewaschenen, adhärenten Zellen werden dann durch Zugabe von Zell-Lyse-Lösung (100 µ/well) und 30-minutiges Erwärmen auf 37°C solubilisiert. Die Zell-Lysate werden in 7-ml-Szintillationsrährchen aus Glas, die 150 µl Wasser enthalten, überführt. Man versetzt mit dem Szintillations-Cocktail (5 ml/Röhrchen), und die mit den Zellen assoziierte Radioaktivität wird flüssigkeitsszintillationsspektroskopisch bestimmt.

Gemäß diesen Assays stellen die Verbindungen der Formel I VEGF-Hemmer dar und eignen sich daher zur Hemmung der Angiogenese, wie bei der Behandlung von Augenkrankheiten, z.B. diabetischer Retinopathie, und zur Behandlung von Karzinomen, z.B. festen Tumoren. Die vorliegenden Verbindungen hemmen die VEGF-stimulierte Mitogenese von kultivierten menschlichen Gefäßendothelzellen mit HK50-Werten von 0,01-5,0 µM. Diese Verbindungen sind im Vergleich zu verwandten Tyrosinkinasen (z.B. FGFR1 sowie Src-Familie; zur Beziehung zwischen Src-Kinasen und VEGFR-Kinasen siehe Eliceiri et al., Molecular Cell, Bd. 4, S.915-924, Dezember 1999) auch selektiv.

Die TIE-2-Tests können z.B. analog der in WO 02/44156 angegebenen Methoden durchgeführt werden.
Der Assay bestimmt die inhibierende Aktivität der zu testenden Substanzen bei der Phosphorylierung des Substrats Poly (Glu, Tyr) durch Tie-2-Kinase in Gegenwart von radioaktivem ³³P-ATP. Das phosphorylierte Substrat bindet während der Inkubationszeit an die Oberfläche einer "flashplate"-Mikrotiterplatte. Nach Entfernen der Reaktionsmischung wird mehrmals gewaschen und anschließend die Radioaktivität an der Oberfläche der Mikrotiterplatte gemessen. Ein inhibierender Effekt der zu messenden Substanzen hat eine geringere Radioaktivität, verglichen mit einer ungestörten enzymatischen Reaktion, zur Folge.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

Massenspektrometrie (MS):
EI (Elektronenstoß-Ionisation) M⁺
FAB (Fast Atom Bombardment) (M+H)⁺
ESI (Electrospray lonization) (M+H)⁺ (wenn
nichts anderes angegeben)

### Beispiel 1

### Synthese von 3-[2-Amino-1-(4-methoxy-benzyl)-1H-benzimidazol-5-yl]-propionsäure (A) und 3-[2-Amino-1-(4-methoxy-benzyl)-1H-benzimidazol-5-yl]-propan-1-ol (B)

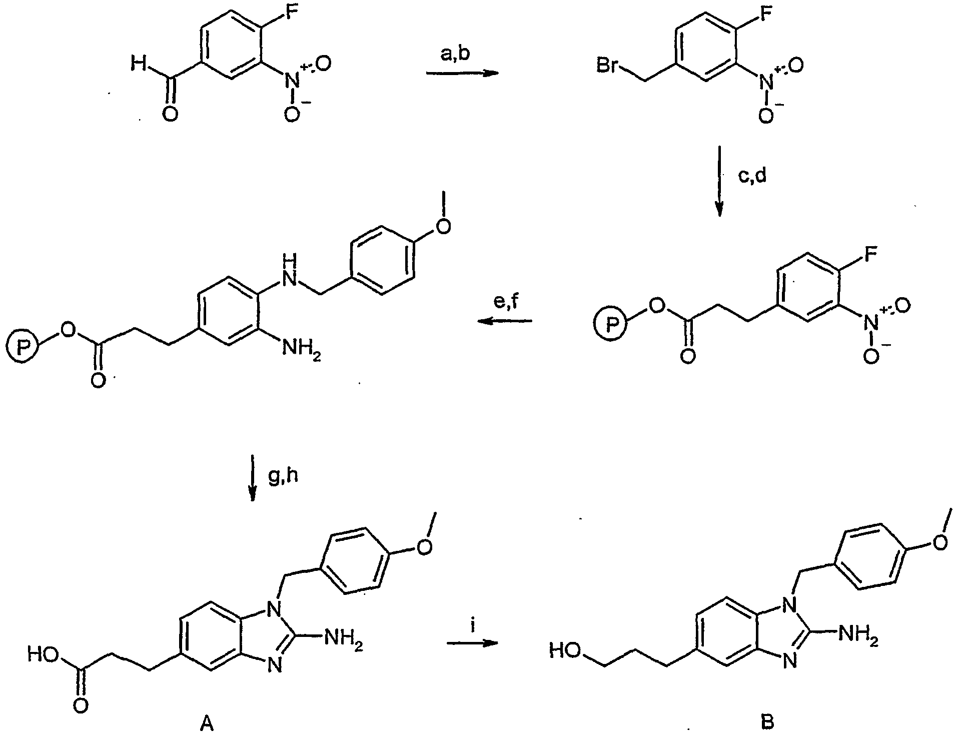

### a

1,69 g (0.01 mol) 4-Fluor-3-nitrobenzaldehyd werden unter Argon in 100 ml n-Heptan suspendiert. Dann gibt man 20 g Kieselgel 60 (0.063-0.200 mm) und 0.378 g (0.01 mol) Natriumborhydrid (Feingranulat, zur Synthese) zu und rührt 90 Minuten bei 40°C. Danach wird abfiltriert und die organische Phase zum Rückstand eingedampft (im Vakuum). Zurück bleiben 1,7 g 4-Fluor-3-nitrobenzylalkohol,

### b

1.3 g (7.6 mmol) 4-Fluor-3-nitrobenzylalkohol werden in 20 ml Dichlormethan gelöst. Man gibt 2.49 ml Bromtrimethylsilan zur Synthese hinzu und rührt über Nacht bei Raumtemperatur. Das Reaktionsgemisch wird im Vakuum zum Rückstand eingedampft und über eine Kieselgelsäule (Laufmittel PE/EE 4:1) aufgereinigt. Man erhält 1.7 g 4-Fluor-3-nitrobenzylbromid.

### c

4.2 g Natriumhydrid (60%ige Suspension in Paraffinöl) zur Synthese werden in 150 ml THF unter Argon vorgelegt. Man tropft eine Mischung von 16.02 ml Diethylmalonat in 50 ml THF hinzu und rührt 5 Minuten bei Raumtemperatur. Dann löst man 5.0 g 4-Fluor-3-nitrobenzylbromid in 50 ml THF und tropft diese ebenfalls zu. Man rührt 15 Minuten bei Raumtemperatur. Das Reaktionsgemisch wird mit je 200 ml Dichlormethan und Wasser verdünnt und die organische Phase abgetrennt. Man wäscht nochmals mit Wasser und trocknet sie mit Magnesiumsulfat. Nach Filtration wird im Vakuum zum Rückstand eingedampft. Man nimmt den Rückstand in 80 ml konz. Salzsäure auf und kocht über Nacht am Rückflusskühler. Die Lösung wird abgekühlt und mit 150 ml Essigester extrahiert. Die Essigesterphase wird mit Magnesiumsulfat getrocknet und im Vakuum zum Rückstand eingedampft. Der Rückstand wird über eine Kieselgelsäule aufgereinigt (Laufmittel EE/PE 1:5, später EE). Man erhält 3.6 g 3-(4-Fluor-3-nitrophenyl)-propionsäure.

### d

4.0 g 3-(4-Fluor-3-nitrophenyl)-propionsäure wird 2 Stunden bei Raumtemperatur in 50 ml Dichlormethan mit 4 ml Thionylchlorid gerührt,dann im Vakuum zum Rückstand eingeengt. Man suspendiert 10 g Wang-Harz in 250 ml Dichlormethan und 3.23 ml N-Diisopropyl-ethylamin. Unter Kühlen im Eisbad tropft man das Säurechlorid hinzu. Dann rührt man 24 Stunden bei Raumtemperatur. Die Reaktionslösung wird abfiltriert und die feste Phase mit jeweils 200 ml Dichlormethan, Dimethylformamid (DMF), DMF/Wasser, DMF, Dichlormethan und Methanol gewaschen und im Vakuum getrocknet. Man erhält 12.4 g polymergebundene 3-(4-Fluor-3-nitrophenyl)-propionsäure.

### e

0.5 g polymergebundene 3-(4-Fluor-3-nitrophenyl)-propionsäure werden in 5 ml DMF suspendiert und mit 1.21 g 4-Methoxybenzylamin und 1.92 ml N-Diisopropyl-ethylamin über Nacht bei Raumtemperatur gerührt. Man filtriert die Reaktionslösung ab und wäscht die feste Phase mit Dichlormethan, Dimethylformamid (DMF), DMF/Wasser, DMF, Dichlormethan und Methanol und trocknet im Vakuum. Man erhält 0.52 g polymergebundene 3-(4- Methoxybenzylamino -3-nitrophenyl)-propionsäure.

### f

0.5 g polymergebundene 3-(4- Methoxybenzylamino -3-nitrophenyl)-propionsäure werden in 4 ml DMF und 1 ml Ethanol suspendiert und mit 2.55 g Zinn(II)chlorid-dihydrat versetzt. Über Nacht wird bei 50°C gerührt. Dann filtriert man vom Reaktionsgemisch ab und wäscht 3 x mit DMF, 2 x mit DMF/Wasser (1:1), 3 x mit DMF, 3 x mit Dichlormethan und 3 x mit Methanol. Man erhält 0.5 g polymergebundene 3-(3-Amino-4-benzylamino)-propion-säure.

### g

0.4 g polymergebundene 3-(3-Amino-4- methoxybenzylamino -)-propionsäure werden in 4 ml DMF und 2 ml Ethanol suspendiert und mit 0.48 g Bromcyan versetzt. Über Nacht wird bei Raumtemperatur gerührt. Dann filtriert man vom Reaktionsgemisch ab und wäscht 3 x mit DMF, 2 x mit DMF/Wasser (1:1), 3 x mit DMF, 3 x mit Dichlormethan und 3 x mit Methanol. Man erhält 0.4 g polymergebundene 3-[2-Amino-1-(4-methoxy-benzyl)-1H-benzimidazol-5-yl]-propionsäure.

### h

0.2 g polymergebundene 3-(2-Amino-1-4-methoxy-1H-benzimidazol-5-yl)-propionsäure werden in 2 ml Trifluoressigsäure/Dichlormethan (1:1) 30 Minuten gerührt. Die Abspaltungslösung wird im Vakuum eingedampft. Das so erhaltene Rohprodukt wird mittels präparativer HPLC über eine RP-18-Säule aufgereinigt. Man erhält 18 mg 3-[2-Amino-1-(4-methoxy-benzyl)-1H-benzimidazol-5-yl]-propionsäure (A).

### i

0.4 g polymergebundene 3-[2-Amino-1-(4-methoxybenzyl)-1H-benzimidazol-5-yl]-propionsäure werden mit 3 ml Toluol versetzt und unter Argonatmosphäre im Eisbad eingekühlt.
3.0 ml Diisobutylaluminiumhydrid (20 % in Toluol)werden zugegeben und bei Raumtemperatur über Nacht stehen gelassen. Die feste Phase wird abfiltriert und gewaschen: 2 x mit Toluol, 2 x mit THF, 1 x mit Wasser/THF(1:1) und 2 x mit Methanol.
Zu den vereinigten organischen Phasen wird nun 1 molare HCl zugegeben bis sich der entstandene Niederschlag wieder löst. Die Lösung wird mit etwas Wasser verdünnt und 4 mal mit Dichlormethan gewaschen. Die vereinigten organischen Phasen werden über Magnesiumsulfat getrocknet, abfiltriert und zum Rückstand eingeengt. Über eine präparative HPLC aufgereinigt erhält man 18.5 mg 3-[2-Amino-1-(4-methoxy-benzyl)-1H-benzimidazol-5-yl]-propan-1-ol (B).

### Beispiel 2

### Synthese von 1-Benzyl-5-morpholin-4-ylmethyl-1 H-benzimidazol-2-ylamin.

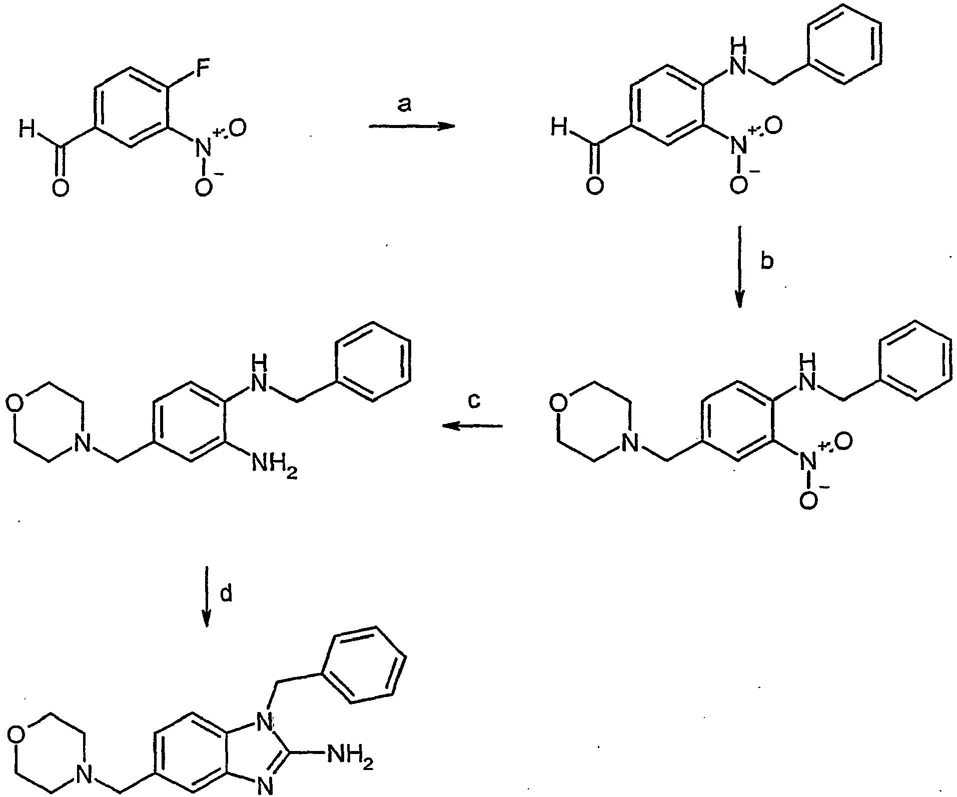

### a

0.2 g 4-Fluor-3-nitro-benzaldehyd und 30 ml Benzylamin werden in DMF gelöst und über Nacht bei Raumtemperatur gerührt. Dann wird das Reaktionsgemisch im Vakuum eingedampft und über eine Kieselgelsäule aufgereinigt. Man erhält 15 g 4-Benzylamino-3-nitro-benzaldehyd.

### b

0.77 g 4-Benzylamino-3-nitro-benzaldehyd und 1.05 ml Morpholin werden in 8 ml absolutem Methanol gelöst. Eine Lösung von 0.19 g Natriumcyanoborhydrid und 0.2 g Zink(II)chlorid in 5 ml absolutem Methanol wird hinzugegeben. Man rührt 2 Stunden bei Raumtemperatur. Dann wird mit 20 ml 0,1 M NaOH versetzt und das Methanol im Vakuum eingedampft. Die wässrige Phase wird mit Essigester extrahiert (3 mal 30 ml). Die vereinigten organischen Phasen werden mit Wasser und gesättigter Natriumchlorid-Lösung extrahiert, mit MgSO4 getrocknet, abfiltriert und im Vakuum eingedampft. Das Rohprodukt (0,8 g) wurde über eine Kieselgelsäule (Laufmittel: PE/EE 1:1) gereinigt. Man erhält 0.6 g Benzyl-(4-morpholin-4-ylmethyl-2-nitro-phenyl)-amin.

### c

0.57 g Benzyl-(4-morpholin-4-ylmethyl-2-nitro-phenyl)-amin werden in Essigester gelöst und mit 2.03 g Zinn(II)chlorid-dihydrat versetzt. Es wird über Nacht am Rückflusskühler gekocht. Die Lösung wurde mit 2,5 M NaOH auf pH 9 eingestellt und mit Essigester extrahiert. Die organische Phase wurde mit Magnesiumsulfat getrocknet, abfiltriert und im Vakuum eingedampft. Das Rohprodukt (0,5 g) wurde über eine Kieselgelsäule (Laufmittel: PE/EE 1:3) gereinigt. Man erhält 60 mg N1-Benzyl-4-morpholin-4-ylmethylphenyl-1,2-diamin.

### d

60 mg N1-Benzyl-4-morpholin-4-ylmethylphenyl-1,2-diamin und 64.2 mg Bromcyan werden in 10 ml Ethanol/DMF 1:2 gelöst und über Nacht bei Raumtemperatur gerührt. Die organische Phase wird im Vakuum eingedampft. Das Rohprodukt wurde über eine Kieselgelsäule (Laufmittel: PE/EE 1:3) gereinigt. Man erhält 9.3 mg 1-Benzyl-5-morpholin-4-ylmethyl-1 H-benzimidazol-2-ylamin.

### Analog erhält man nachstehende Verbindungen:

| VERBINDUNGEN | MOLEKULARGEWICHT | Retentionszeit [min] |
|---|---|---|
| 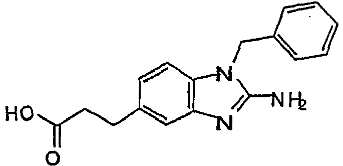 | 295,3 | 2,45 |
| 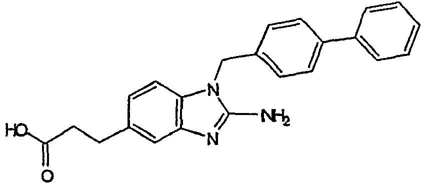 | 371,4 | 3,33 |
| 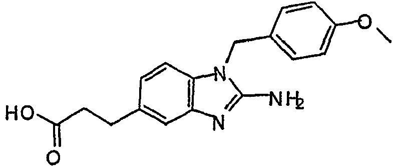 | 325,4 | 2,67 |
| 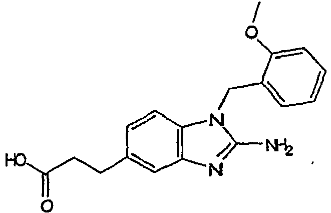 | 325,4 | 3,31 |
| 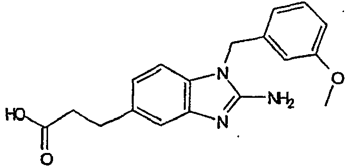 | 325,4 | 2,67 |
| 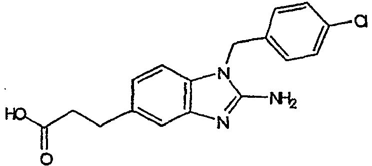 | 329,8 | 2,91 |
| 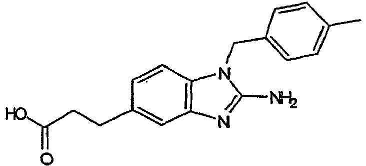 | 309,4 | 2,80 |
| 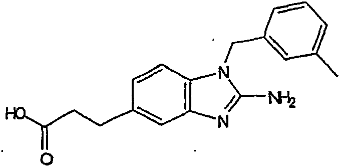 | 309,4 | 2,85 |
| 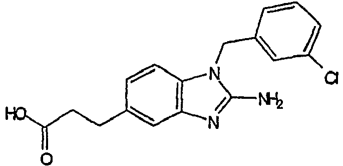 | 329,8 | 2,85 |
| 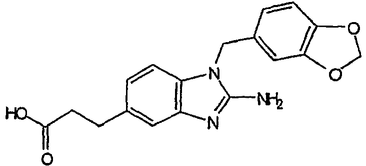 | 339,3 | 2,64 |
| 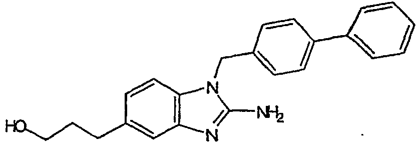 | 357,5 | 3,25 |
| 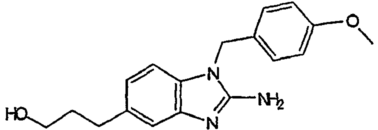 | 311,4 | 2,59 |
| 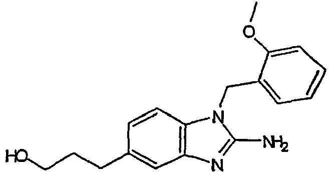 | 311,4 | 2,72 |
| 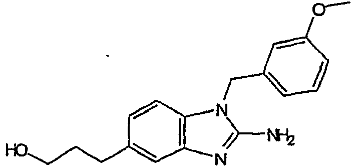 | 311,4 | 2,67 |
| 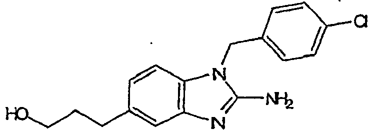 | 315,8 | 2,88 |
| 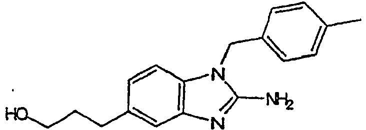 | 295,4 | 2,8 |
| 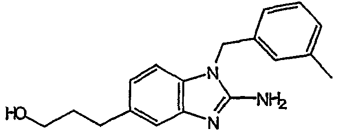 | 295,4 | 2,75 |
| 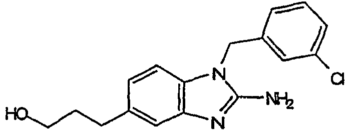 | 315,8 | 2,88 |
| 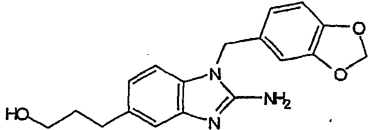 | 325,4 | 2,61 |
| 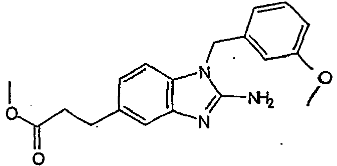 | 339,4 | 3,04 |
| 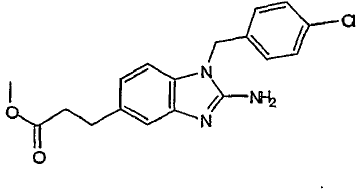 | 343,8 | 3,28 |
| 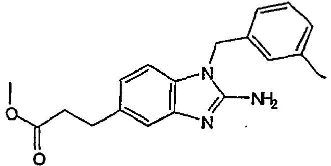 | 323,4 | 3,17 |
| 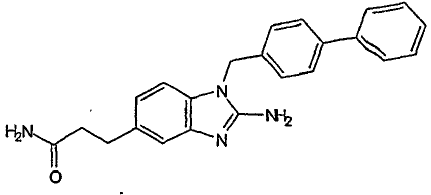 | 370,5 | 3,09 |
| 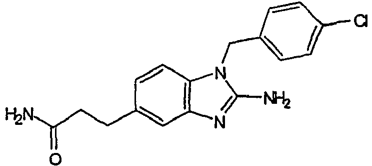 | 328,8 | 2,67 |
| 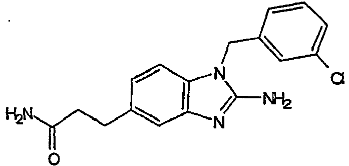 | 328,8 | 2,61 |
| 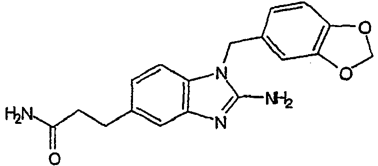 | 338,4 | 2,35 |
| 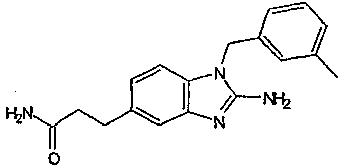 | 308,4 | 2,59 |
| 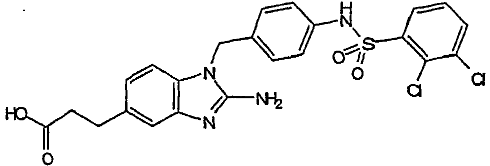 | 519,4 | 3,12 |
| 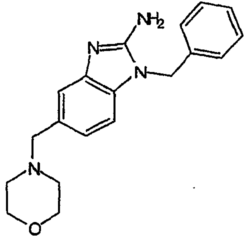 | 322,4 | 2,08 |
| 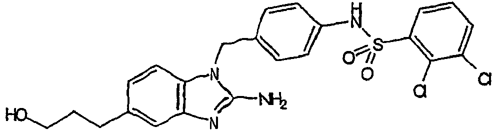 | 505,4 | 3,2 |
| 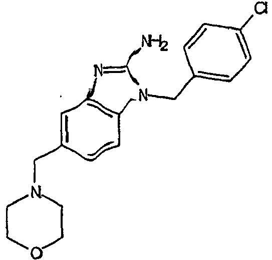 | 356,9 | 2,24 |
| 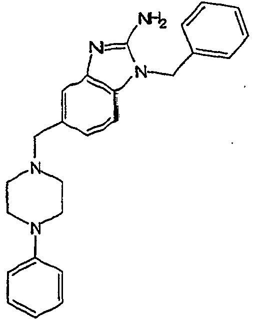 | 397,5 | 2,61 |
| 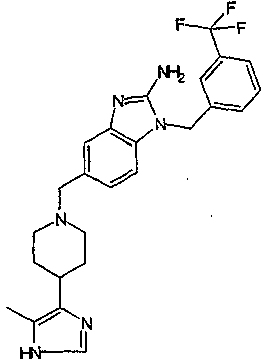 | 468,5 | 2,27 |
| 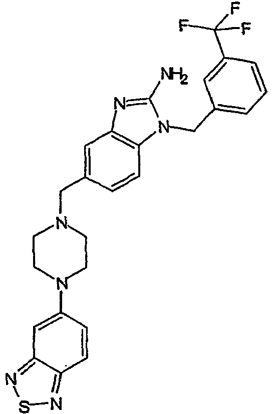 | 523,6 | 2,96 |
| 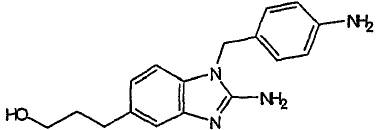 | 296,4 | 1,6 |
| 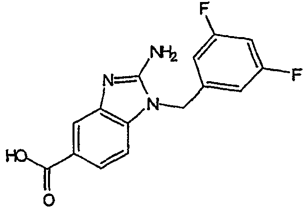 | 303,3 | 2,51 |
| 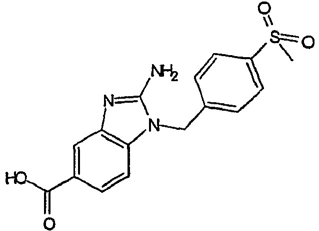 | 345,4 | 1,92 |
| 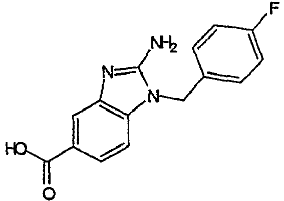 | 285,3 | 2,43 |

### Pharmakologische Testergebnisse

| VERBINDUNG | Inhibierung von TIE-2 IC₅₀ (nMol) |
|---|---|
| N-[4-(2-Amino-5-bromo-benzimidazol-1-ylmethyl)-phenyl]-2,3-dichlor-benzenesulfonamid | 2780 |
| N-{4-[2-Amino-5-(3-hydroxy-propyl)-benzimidazol-1-ylmethyl]-phenyl}-2,3-dichlor-benzolsulfonamid | 320 |

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38g NaH₂PO₄ · 2 H₂O, 28,48 g Na₂HPO₄ ·12H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

### Eine Lösung von 1 kg eines

erfindungsgemäßen Wirkstoffes in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen, ausgewählt aus der Gruppe
3-((2-Amino-1-benzyl-1H-benzimidazol-5-yl)-propionsäure,
3-((2-Amino-1-biphenyl-4-ylmethyl-1H-benzimidazol-5-yl)-propionsäure,
3-[2-Amino-1-(4-methoxy-benzyl)-1H-benzimidazol-5-yl]-propionsäure,
3-[2-Amino-1-(2-methoxy-benzyl)-1H-benzimidazol-5-yl]-propionsäure,
3-[2-Amino-1-(3-methoxy-benzyl)-1H-benzimidazol-5-yl]-propionsäure,
3-[2-Amino-1-(4-chlor-benzyl)-1H-benzimidazol-5-yl]-propionsäure,
3-[2-Amino-1-(4-methyl-benzyl)-1H-benzimidazol-5-yl]-propionsäure,
3-[2-Amino-1-(3-methyl-benzyl)-1H-benzimidazal-5--yl]-propionsäure,
3-[2-Amino-1-(3-chlor-benzyl)-1H-benzimidazol-5-yl]-propionsäure,
3-((2-Amino-1-benzo[1,3]dioxol-5-ylmethyl-1H-benzimidazol-5-yl)-propionsäure,
3-((2-Amino-1-biphenyl-4-ylmethyl-1H-benzimidazol-5-yl)-propan-1-ol,
3-[2-Amino-1-(4-methoxy-benzyl)-1H-benzimidazol-5-yl]-propan-1-ol,
3-[2-Amino-1-(2-methoxy-benzyl)-1H-benzimidazol-5-yl]-propan-1-ol,
3-[2-Amino-1-(3-methoxy-benzyl)-1H-benzimidazol-5-yl]-propan-1-ol,
3-[2-Amino-1-(4-chlor-benzyl)-1H-benzimidazol-5-yl]-propan-1-ol,
3-[2-Amino-1-(4-methyl-benzyl)-1H-benzimidazol-5-yl]-propan-1-ol,
3-[2-Amino-1-(3-methyl-benzyl)-1H-benzimidazol-5-yl]-propan-1-ol,
3-[2-Amino-1-(3-chlor-benzyl)-1H-benzimidazol-5-yl]-propan-1-ol,
3-((2-Amino-1-benzo[1,3]dioxol-5-ylmethyl-1H-benzimidazol-5-yl)-propan-1-ol,
3-[2-Amino-1-(3-methoxy-benzyl)-1H-benzimidazol-5-yl]-propionsäure methyl ester,
3-[2-Amino-1-(4-chlor-benzyl)-1H-benzimidazol-5-yl]-propionsäure methyl ester,
3-[2-Amino-1-(3-methyl-benzyl)-1H-benzimidazol-5-yl]-propionsäure methyl ester,
3-((2-Amino-1-biphenyl-4-ylmethyl-1H-benzimidazol-5-yl)-propionamid,
3-[2-Amino-1-(4-chlor-benzyl)-1H-benzimidazol-5-yl]-propionamid
3-((2-Amino-1-benzo[1,3]dioxol-5-ylmethyl-1H-benzimidazol-5-yl)-propionamid,
3-[2-Amino-1-(3-methyl-benzyl)-1H-benzimidazol-5-yl]-propionamid,
3-{2-Amino-1-[4-(2,3-dichlor-benzolsulfonylamino)-benzyl]-1H-benzimidazol-5-yl}-propionsäure,
1-Benzyl-5-morpholin-4-ylmethyl-1H-benzimidazol-2-ylamine,
N-{4-[2-Amino-5-(3-hydroxy-propyl)-benzimidazol-1-ylmethyl]-phenyl}-2,3-dichlor-benzolsulfonamid,
1-((4-Chlor-benzyl)-5-morpholin-4-ylmethyl-1H-benzimidazol-2-ylamin,
1-Benzyl-5-(4-phenyl-piperazin-1-ylmethyl)-1H-benzimidazol-2-ylamin,
5-[4-(5-Methyl-1H-imidazol-4-yl)-piperidin-1-ylmethyl]-1-(3-trifluoromethyl-benzyl)-1H-benzimidazol-2-ylamin,
5-((4-Benzo[1,2,5]thiadiazol-5-yl-piperazin-1-ylmethyl)-1-(3-trifluoromethyl-benzyl)-1H-benzimidazol-2-ylamin,
3-[2-Amino-1-(4-amino-benzyl)-1H-benzimidazol-5-yl]-propan-1-ol,
2-Amino-1-(3,5-difluor-benzyl)-1H-benzimidazol-5-carbon säure,
2-Amino-1-(4-methanesulfonyl-benzyl)-1H-benzimidazol-5-carbonsäure,
2-Amino-1-(4-fluor-benzyl)-1H-benzimidazol-5-carbonsäure,
sowie ihre Solvate und Stereoisomere, einschließlich deren Mischungen in allen Verhältnissen.

2. Arzneimittel, enthaltend mindestens eine Verbindung nach Anspruch 1 und/oder ihre Solvate und Stereoisomere, einschließlich deren Mischungen in allen Verhältnissen, sowie gegebenenfalls Träger- und/oder Hilfsstoffe als Tyrosinkinase Modulator.

3. Verwendung von Verbindungen nach Anspruch 1, sowie ihrer Solvate und Stereoisomere, einschließlich deren Mischungen in allen Verhältnissen, zur Herstellung eines Arzneimittels zur Behandlung und/oder Prophylaxe von Krankheiten, bei denen die Hemmung, Regulierung und/oder Modulation der Signaltransduktion von Kinasen eine Rolle spielt.

4. Verwendung nach Anspruch 3, wobei die Kinasen ausgewählt sind aus der Gruppe der Tyrosinkinasen.

5. Verwendung nach Anspruch 4, wobei es sich bei den Tyrosinkinasen um TIE-2, VEGFR, PDGFR, FGFR und/oder FLT/KDR handelt.

6. Verwendung nach Anspruch 4 von Verbindungen gemäß Anspruch 1, sowie ihrer Solvate und Stereoisomere, einschließlich deren Mischungen in allen Verhältnissen, zur Herstellung eines Arzneimittels zur Behandlung von Krankheiten, die durch Inhibierung der Tyrosinkinasen durch die Verbindungen nach Anspruch 1 beeinflußt werden.

7. Verwendung nach Anspruch 5, zur Herstellung eines Arzneimittels zur Behandlung von Krankheiten, die durch Inhibierung von TIE-2, VEGFR, PDGFR, FGFR und/oder FLT/KDR durch die Verbindungen nach Anspruch 1 beeinflußt werden.

8. Verwendung nach Anspruch 6 oder 7 wobei die zu behandelnde Krankheit ein Tumor ist.

9. Verwendung nach Anspruch 8, wobei der Tumor, ein fester Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Kehlkopf und/oder der Lunge stammt.

10. Verwendung nach Anspruch 8, wobei der feste Tumor aus der Gruppe der Lungenadenokarzinom, kleinzellige Lungenkarzinome, Bauchspeicheldrüsenkrebs, Glioblastome, Kolonkarzinom und Brustkarzinom stammt.

11. Verwendung nach Anspruch 6 oder 7, wobei die zu behandelnde Krankheit ein Tumor des Blut- und Immunsystems ist.

12. Verwendung nach Anspruch 11, wobei der Tumor aus der Gruppe der akuten myelotischen Leukämie, der chronischen myelotischen Leukämie, akuten lymphatischen Leukämie und/oder chronischen lymphatischen Leukämie stammt.

13. Verwendung nach Anspruch 6 oder 7 zur Behandlung einer Krankheit, an der Angiogenese beteiligt ist.

14. Verwendung nach Anspruch 13, wobei es sich bei der Krankheit um eine Augenkrankheit handelt.

15. Verwendung nach Anspruch 14 zur Behandlung von Retina-Vaskularisierung, diabetischer Retinopathie und/oder altersbedingter Makula-Degeneration.

16. Verwendung nach Anspruch 6 oder 7 zur Behandlung von Knochen-Pathologien, wobei die Knochenpathologie aus der Gruppe Osteosarkom, Osteoarthritis und Rachitis stammt.

17. Arzneimittel enthaltend mindestens eine Verbindung gemäß Anspruch 1 und/oder ihre Solvate und Stereoisomere, einschließlich deren Mischungen in allen Verhältnissen, und mindestens einen weiteren Arzneimittelwirkstoff.

18. Set (Kit), bestehend aus getrennten Packungen von
(a) einer wirksamen Menge an einer Verbindung gemäß Anspruch 1 und/oder ihrer Solvate und Stereoisomere, einschließlich deren Mischungen in allen Verhältnissen,
und
(b) einer wirksamen Menge eines weiteren Arzneimittetswirkstoffs.

19. Verwendung von Verbindungen gemäß Anspruch 1 und/oder ihrer physiologisch unbedenklichen Salze und Solvate zur Herstellung eines Arzneimittels zur Behandlung von festen Tumoren, wobei eine therapeutisch wirksame Menge einer Verbindung nach Anspruch 1 in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

20. Verwendung von Verbindungen nach Anspruch 1 und/oder ihrer physiologisch unbedenklichen Salze und Solvate zur Herstellung eines Arzneimittels zur Behandlung von festen Tumoren wobei eine therapeutisch wirksame Menge einer Verbindung nach Anspruch 1 in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

21. Verwendung nach Anspruch 3, 4 oder 5, zur Herstellung eines Arzneimittels zur Behandlung von Krankheiten, die auf einer gestörten TIE-2-Aktivität beruhen,
wobei eine therapeutisch wirksame Menge einer Verbindung nach Anspruch 1 in Kombination mit einem Wachstumsfaktorrezeptor-Hemmer verabreicht wird.

22. Verwendung nach Anspruch 3, 4 oder 5 zur Behandlung von Erkrankungen ausgewählt sind aus der Gruppe der hyperproliferativen und nicht hyperproliferativen Erkrankungen die nicht zu den krebsartigen Erkrankungen gehören.

23. Verwendung nach Anspruch 22, wobei die nicht krebsartigen Erkrankungen ausgewählt sind aus der Gruppe bestehend aus Psoriasis, Arthritis, Entzündungen, Kontaktdermatitis und Spät-Typ der Überempfindlichkeitsreaktion, Endometriose, Vernarbung, gutartiger Prostatahyperplasie, immunologischer Krankheiten, Autoimmunkrankheiten und Immunschwächekrankheiten.

## Claims

1. Compounds selected from the group
3-((2-amino-1-benzyl-1H-benzimidazol-5-yl)propionic acid,
3-((2-amino-1-biphenyl-4-ylmethyl-1H-benzimidazol-5-yl)propionic acid,
3-[2-amino-1-(4-methoxybenzyl)-1H-benzimidazol-5-yl]propionic acid,
3-[2-amino-1-(2-methoxybenzyl)-1H-benzimidazol-5-yl]propionic acid,
3-[2-amino-1-(3-methoxybenzyl)-1H-benzimidazol-5-yl]propionic acid,
3-[2-amino-1-(4-chlorobenzyl)-1H-benzimidazol-5-yl]propionic acid,
3-[2-amino-1-(4-methylbenzyl)-1H-benzimidazol-5-yl]propionic acid,
3-[2-amino-1-(3-methylbenzyl)-1H-benzimidazol-5-yl]propionic acid,
3-[2-amino-1-(3-chlorobenzyl)-1H-benzimidazol-5-yl]propionic acid,
3-((2-amino-1-benzo-1,3-dioxol-5-ylmethyl-1H-benzimidazol-5-yl)-propionic acid,
3-((2-amino-1-biphenyl-4-ylmethyl-1H-benzimidazol-5-yl)propan-1-ol,
3-[2-amino-1-(4-methoxybenzyl)-1H-benzimidazol-5-yl]propan-1-ol,
3-[2-amino-1-(2-methoxybenzyl)-1H-benzimidazol-5-yl]propan-1-ol,
3-[2-amino-1-(3-methoxybenzyl)-1H-benzimidazol-5-yl]propan-1-ol,
3-[2-amino-1-(4-chlorobenzyl)-1H-benzimidazol-5-yl]propan-1-ol,
3-[2-amino-1-(4-methylbenzyl)-1H-benzimidazol-5-yl]propan-1-ol,
3-[2-amino-1-(3-methylbenzyl)-1H-benzimidazol-5-yl]propan-1-ol,
3-[2-amino-1-(3-chlorobenzyl)-1H-benzimidazol-5-yl]propan-1-ol,
3-((2-amino-1-benzo-1,3-dioxol-5-ylmethyl-1H-benzimidazol-5-yl)propan-1-ol,
3-[2-amino-1-(3-methoxybenzyl)-1H-benzimidazol-5-yl]propionic acid methyl ester,
3-[2-amino-1-(4-chlorobenzyl)-1H-benzimidazol-5-yl]propionic acid methyl ester,
3-[2-amino-1-(3-methylbenzyl)-1H-benzimidazol-5-yl]propionic acid methyl ester,
3-((2-amino-1-biphenyl-4-ylmethyl-1H-benzimidazol-5-yl)propionamide,
3-[2-amino-1-(4-chlorobenzyl)-1H-benzimidazol-5-yl]propionamide,
3-((2-amino-1-benzo-1,3-dioxol-5-ylmethyl-1H-benzimidazol-5-yl)-propionamide,
3-[2-amino-1-(3-methylbenzyl)-1H-benzimidazol-5-yl]propionamide,
3-{2-amino-1-[4-(2,3-dichlorobenzenesulfonylamino)benzyl]-1H-benzimidazol-5-yl}propionic acid,
1-benzyl-5-morpholin-4-ylmethyl-1H-benzimidazol-2-ylamine,
N-{4-[2-amino-5-(3-hydroxypropyl)benzimidazol-1-ylmethyl]phenyl}-2,3-dichlorobenzenesulfonamide,
1-((4-chlorobenzyl)-5-morpholin-4-ylmethyl-1H-benzimidazol-2-yl-amine,
1-benzyl-5-(4-phenylpiperazin-1-ylmethyl)-1H-benzimidazol-2-yl-amine,
5-[4-(5-methyl-1H-imidazol-4-yl)piperidin-1-ylmethyl]-1-(3-trifluoromethylbenzyl)-1H-benzimidazol-2-ylamine,
5-((4-benzo-1,2,5-thiadiazol-5-ylpiperazin-1-ylmethyl)-1-(3-trifluoromethylbenzyl)-1H-benzimidazol-2-ylamine,
3-[2-amino-1-(4-aminobenzyl)-1H-benzimidazol-5-yl]propan-1-ol,
2-amino-1-(3,5-difluorobenzyl)-1H-benzimidazole-5-carboxylic acid,
2-amino-1-(4-methanesulfonylbenzyl)-1H-benzimidazole-5-carboxylic acid,
2-amino-1-(4-fluorobenzyl)-1H-benzimidazole-5-carboxylic acid,
and solvates and stereoisomers thereof, including mixtures thereof in all ratios.

2. Medicaments comprising at least one compound according to Claim 1 and/or solvates and stereoisomers thereof, including mixtures thereof in all ratios, and optionally excipients and/or adjuvants, as tyrosine kinase modulator.

3. Use of compounds according to Claim 1, and solvates and stereoisomers thereof, including mixtures thereof in all ratios, for the preparation of a medicament for the treatment and/or prophylaxis of diseases in which the inhibition, regulation and/or modulation of kinase signal transduction plays a role.

4. Use according to Claim 3, where the kinases are selected from the group of tyrosine kinases.

5. Use according to Claim 4, where the tyrosine kinases are TIE-2, VEGFR, PDGFR, FGFR and/or FLT/KDR.

6. Use according to Claim 4 of compounds according to Claim 1, and solvates and stereoisomers thereof, including mixtures thereof in all ratios, for the preparation of a medicament for the treatment of diseases which are influenced by inhibition of tyrosine kinases by the compounds according to Claim 1.

7. Use according to Claim 5 for the preparation of a medicament for the treatment of diseases which are influenced by inhibition of TIE-2, VEGFR, PDGFR, FGFR and/or FLT/KDR by the compounds according to Claim 1.

8. Use according to Claim 6 or 7, where the disease to be treated is a tumour.

9. Use according to Claim 8, where the tumour is a solid tumour from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the larynx and/or of the lung.

10. Use according to Claim 8, where the solid tumour originates from the group of lung adenocarcinoma, small-cell lung carcinomas, pancreatic cancer, glioblastomas, colon carcinoma and breast carcinoma.

11. Use according to Claim 6 or 7, where the disease to be treated is a tumour of the blood and immune system.

12. Use according to Claim 11, where the tumour originates from the group of acute myeloid leukaemia, chronic myeloid leukaemia, acute lymphatic leukaemia and/or chronic lymphatic leukaemia.

13. Use according to Claim 6 or 7 for the treatment of a disease in which angiogenesis is implicated.

14. Use according to Claim 13, where the disease is an eye disease.

15. Use according to Claim 14 for the treatment of retinal vascularisation, diabetic retinopathy and/or age-induced macular degeneration.

16. Use according to Claim 6 or 7 for the treatment of bone pathologies, where the bone pathology originates from the group osteosarcoma, osteoarthritis and rickets.

17. Medicaments comprising at least one compound according to Claim 1 and/or solvates and stereoisomers thereof, including mixtures thereof in all ratios, and at least one further medicament active compound.

18. Set (kit) consisting of separate packs of
(a) an effective amount of a compound according to Claim 1 and/or solvates and stereoisomers thereof, including mixtures thereof in all ratios,
and
(b) an effective amount of a further medicament active compound.

19. Use of compounds according to Claim 1 and/or physiologically acceptable salts and solvates thereof for the preparation of a medicament for the treatment of solid tumours, where a therapeutically effective amount of a compound according to Claim 1 is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) other angiogenesis inhibitors.

20. Use of compounds according to Claim 1 and/or physiologically acceptable salts and solvates thereof for the preparation of a medicament for the treatment of solid tumours, where a therapeutically effective amount of a compound according to Claim 1 is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) other angiogenesis inhibitors.

21. Use according to Claim 3, 4 or 5 for the preparation of a medicament for the treatment of diseases which are based on disturbed TIE-2 activity,
where a therapeutically effective amount of a compound according to Claim 1 is administered in combination with a growth-factor receptor inhibitor.

22. Use according to Claim 3, 4 or 5 for the treatment of diseases selected from the group of hyperproliferative and non-hyperproliferative diseases which do not belong to the cancerous diseases.

23. Use according to Claim 22, where the non-cancerous diseases are selected from the group consisting of psoriasis, arthritis, inflammation, contact dermatitis and delayed hypersensitivity reaction, endometriosis, scarring, benign prostatic hyperplasia, immunological diseases, autoimmune diseases and immunodeficiency diseases.

## Revendications

1. Composés choisis dans le groupe constitué par
l'acide 3-(2-amino-1-benzyl-1 H-benzimidazol-5-yl)propionique,
l'acide 3-(2-amino-1-biphényl-4-ylméthyl-1 H-benzimidazol-5-yl)-propionique,
l'acide 3-[2-amino-1-(4-méthoxybenzyl)-1H-benzimidazol-5-yl]-propionique,
l'acide 3-[2-amino-1-(2-méthoxybenzyl)-1H-benzimidazol-5-yl]-propionique,
l'acide 3-[2-amino-1-(3-méthoxybenzyl)-1H-benzimidazol-5-yl]-propionique,
l'acide 3-[2-amino-1-(4-chlorobenzyl)-1H-benzimidazol-5-yl]-propionique,
l'acide 3-[2-amino-1-(4-méthylbenzyl)-1H-benzimidazol-5-yl]-propionique,
l'acide 3-[2-amino-1-(3-méthylbenzyl)-1H-benzimidazol-5-yl]-propionique,
l'acide 3-[2-amino-1-(3-chlorobenzyl)-1H-benzimidazol-5-yl]-ropionique,
l'acide 3-(2-amino-1-benzo-1,3-dioxol-5-ylméthyl-1H-benzimidazol-5-yl)propionique,
le 3-(2-amino-1-biphényl-4-ylméthyl-1H-benzimidazol-5-yl)propan-1-ol,
le 3-[2-amino-1-(4-méthoxybenzyl)-1H-benzimidazol-5-yl]propan-1-ol,
le 3-[2-amino-1-(2-méthoxybenzyl)-1H-benzimidazol-5-yl]propan-1-ol,
le 3-[2-amino-1-(3-méthoxybenzyl)-1H-benzimidazol-5-yl]propan-1-ol,
le 3-[2-amino-1-(4-chlorobenzyl)-1H-benzimidazol-5-yl]propan-1-ol,
le 3-[2-amino-1-(4-méthylbenzyl)-1H-benzimidazol-5-yl]propan-1-ol,
le 3-[2-amino-1-(3-méthylbenzyl)-1H-benzimidazol-5-yl]propan-1-ol,
le 3-[2-amino-1-(3-chlorobenzyl)-1H-benzimidazol-5-yl]propan-1-ol,
le 3-(2-amino-1-benzo-1,3-dioxol-5-ylméthyl-1H-benzimidazol-5-yl)-propan-1-ol,
le méthylester de l'acide 3-[2-amino-1-(3-méthoxybenzyl)-1H-benzimidazol-5-yl]propionique,
le méthylester de l'acide 3-[2-amino-1-(4-chlorobenzyl)-1H-benzimidazol-5-yl]propionique,
le méthylester de l'acide 3-[2-amino-1-(3-méthylbenzyl)-1H-benzimidazol-5-yl]propionique,
le 3-(2-amino-1-biphényl-4-ylméthyl-1 H-benzimidazol-5-yl)-propionamide,
le 3-[2-amino-1-(4-chlorobenzyl)-1 H-benzimidazol-5-yl]propionamide,
le 3-(2-amino-1-benzo-1,3-dioxol-5-ylméthyl-1H-benzimidazol-5-yl)-propionamide,
le 3-[2-amino-1-(3-méthylbenzyl)-1 H-benzimidazol-5-yl]propionamide,
l'acide 3-{2-amino-1-[4-(2,3-dichlorobenzènesulfonylamino)benzyl]-1H-benzimidazol-5-yl}propionique,
la 1-benzyl-5-morpholin-4-ylméthyl-1H-benzimidazol-2-ylamine,
le N-{4-[2-amino-5-(3-hydroxypropyl)benzimidazol-1-ylméthyl]phényl}-2,3-dichlorobenzènesulfonamide,
la 1-(4-chlorobenzyl)-5-morpholin-4-ylméthyl-1H-benzimidazol-2-yl-amine,
la 1-benzyl-5-(4-phénylpipérazin-1-ylméthyl)-1H-benzimidazol-2-yl-amine,
la 5-[4-(5-méthyl-1 H-imidazol-4-yl)pipéridin-1-ylméthyl]-1-(3-trifluoro-méthylbenzyl)-1H-benzimidazol-2-ylamine,
la 5-(4-benzo-1,2,5-thiadiazol-5-ylpipérazin-1-ylméthyl)-1-(3-trifluoro-méthylbenzyl)-1H-benzimidazol-2-ylamine,
le 3-[2-amino-1-(4-aminobenzyl)-1H-benzimidazol-5-yl]propan-1-ol,
l'acide 2-amino-1-(3,5-difluorobenzyl)-1H-benzimidazole-5-carboxylique,
l'acide 2-amino-1-(4-méthanesulfonylbenzyl)-1 H-benzimidazole-5-carboxylique,
l'acide 2-amino-1-(4-fluorobenzyl)-1H-benzimidazole-5-carboxylique,
et solvats et stéréoisomères de ceux-ci, y compris des mélanges de ceux-ci en toutes proportions.

2. Médicaments comprenant au moins un composé selon la revendication 1 et/ou des solvats et stéréoisomères de celui-ci, y compris des mélanges de ceux-ci en toutes proportions, et éventuellement des excipients et/ou des adjuvants, comme modulateur de tyrosine kinase.

3. Utilisation de composés selon la revendication 1, et de solvats et stéréoisomères de ceux-ci, y compris des mélanges de ceux-ci en toutes proportions, pour la préparation d'un médicament destiné au traitement et/ou à la prophylaxie de maladies dans lesquelles l'inhibition, la régulation et/ou la modulation de la transduction des signaux de kinases joue un rôle.

4. Utilisation selon la revendication 3, dans laquelle les kinases sont choisies parmi le groupe constitué par les tyrosine kinases.

5. Utilisation selon la revendication 4, dans laquelle les tyrosine kinases sont TIE-2, VEGFR, PDGFR, FGFR et/ou FLT/KDR.

6. Utilisation selon la revendication 4 de composés selon la revendication 1, et de solvats et stéréoisomères de ceux-ci, y compris des mélanges de ceux-ci en toutes proportions, pour la préparation d'un médicament destiné au traitement de maladies qui sont influencées par l'inhibition de tyrosine kinases par les composés selon la revendication 1.

7. Utilisation selon la revendication 5 pour la préparation d'un médicament destiné au traitement de maladies qui sont influencées par l'inhibition de TIE-2, VEGFR, PDGFR, FGFR et/ou FLT/KDR par les composés selon la revendication 1.

8. Utilisation selon la revendication 6 ou 7, dans laquelle la maladie à traiter est une tumeur.

9. Utilisation selon la revendication 8, dans laquelle la tumeur est une tumeur solide provenant du groupe constitué par les tumeurs de l'épithélium squameux, de la vessie, de l'estomac, du rein, de la tête et du cou, de l'oesophage, du col de l'utérus, de la thyroïde, des intestins, du foie, du cerveau, de la prostate, du tractus urogénital, du système lymphatique, de l'estomac, du larynx et/ou du poumon.

10. Utilisation selon la revendication 8, dans laquelle la tumeur solide provient du groupe constitué par l'adénocarcinome du poumon, les carcinomes du poumon à petites cellules, le cancer du pancréas, le carcinome du côlon et le carcinome du sein.

11. Utilisation selon la revendication 6 ou 7, dans laquelle la maladie à traiter est une tumeur du système sanguin et immunitaire.

12. Utilisation selon la revendication 11, dans laquelle la tumeur provient du groupe constitué par la leucémie myéloïde aiguë, la leucémie myéloïde chronique, la leucémie lymphoïde aiguë et/ou la leucémie lymphoïde chronique.

13. Utilisation selon la revendication 6 ou 7, pour le traitement d'une maladie dans laquelle l'angiogenèse est impliquée.

14. Utilisation selon la revendication 13, dans laquelle la maladie est une maladie oculaire.

15. Utilisation selon la revendication 14, destinée au traitement de la vascularisation rétinienne, de la rétinopathie diabétique et/ou de la dégénérescence maculaire liée à l'âge.

16. Utilisation selon la revendication 6 ou 7, destinée au traitement de pathologies osseuses, dans laquelle la pathologie osseuse provient du groupe constitué par l'ostéosarcome, l'arthrose et le rachitisme.

17. Médicaments comprenant au moins un composé selon la revendication 1 et/ou des solvats et stéréoisomères de celui-ci, y compris des mélanges de ceux-ci en toutes proportions, et au moins un autre composé actif médicamenteux.

18. Ensemble (kit) constitué de conditionnements séparés
(a) d'une quantité efficace d'au moins un composé selon la revendication 1 et/ou de solvats et stéréoisomères de celui-ci, y compris des mélanges de ceux-ci en toutes proportions,
et
(b) d'une quantité efficace d'un autre composé actif médicamenteux.

19. Utilisation de composés selon la revendication 1 et/ou de sels et de solvats physiologiquement acceptables de ceux-ci pour la préparation d'un médicament destiné au traitement de tumeurs solides, dans laquelle une quantité thérapeutiquement efficace d'un composé selon la revendication 1 est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

20. Utilisation de composés selon la revendication 1 et/ou de sels et de solvats physiologiquement acceptables de ceux-ci pour la préparation d'un médicament destiné au traitement de tumeurs solides, dans laquelle une quantité thérapeutiquement efficace d'un composé selon la revendication 1 est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

21. Utilisation selon la revendication 3, 4 ou 5, pour la préparation d'un médicament destiné au traitement de maladies basées sur une activité perturbée de TIE-2,
dans laquelle une quantité thérapeutiquement efficace d'un composé selon la revendication 1 est administrée en association avec un inhibiteur du récepteur du facteur de croissance.

22. Utilisation selon la revendication 3, 4 ou 5, destinée au traitement de maladies choisies parmi le groupe constitué par les maladies hyper-prolifératives et non hyperprolifératives n'appartenant pas aux maladies cancéreuses.

23. Utilisation selon la revendication 22, dans laquelle les maladies non cancéreuses sont choisies dans le groupe constitué par le psoriasis, l'arthrite, les inflammations, la dermite de contact et la réaction d'hypersensibilité retardée, l'endométriose, la cicatrisation, l'hyperplasie bénigne de la prostate, les maladies immunologiques, les maladies auto-immunes et les maladies d'immunodéficience.
